# EUROPEAN PATENT APPLICATION

(11) **EP 2 031 059 A1**
(43) Date of publication of application: **04.03.2009**
(21) Application number: 07075750.5
(22) Date of filing: 03.09.2007
(51) Int. Cl.: C12N 15/11, A61K 31/713

(54) **siDNA against hepatites C virus (HCV)**

(71) Applicant: Mölling, Karin, 14195 Berlin (DE)
(72) Inventor: Mölling, Karin, 14195 Berlin (DE)
(74) Representative: Boeckh, Tobias

(57) **Abstract**

Silencing of HCV RNA can be achieved by siDNA. These are oligodeoxynucleotides consisting of an antisense-strand homologous to the viral RNA and a second strand, partially complementary to the antisense-strand. The two strands are preferentially linked by a linker (eg 4 thymidines). Triple-helix formation is a preferred effect. The siDNA is superior to siRNA because the formation of RNA-DNA hybrids is preferred over double-stranded DNA or double-stranded RNA, which forms as tertiary structures in RNA genomes. Also the induction of interferon is less likely. siDNA is easier to synthesize and it is more stable. It can be combined with siRNA.

## Description

Silencing of HCV RNA can be achieved by siDNA. These are oligodeoxynucleotides consisting of an antisense-strand homologous to the viral RNA and a second strand, partially complementary to the antisense-strand. The two strands are preferentially linked by a linker (eg 4 thymidines). Triple-helix formation is a preferred effect. The siDNA is superior to siRNA because the formation of RNA-DNA hybrids is preferred over double-stranded DNA or double-stranded RNA, which forms as tertiary structures in RNA genomes. Also the induction of interferon is less likely. Furthermore, siDNA is effective at earlier time-points after addition to the cell than siRNA. siDNA is easier to synthesize and it is more stable. It can also be combined with other siDNAs targeted against various strains in a cocktail; it can also be combined with siRNA to target early and late steps in viral replication.

Hepatitis C virus (HCV) is a small (about 50 nm in size), enveloped, single-stranded, positive sense RNA virus in the family *Flaviviridae.* At least six HCV genotypes are known, which can be further grouped into various subtypes differing in nucleotide sequence by 20 to 25%. Over 170 million people are persistently infected with HCV, and approximately 38'000 new cases are registered annually in the United States alone (CDC Fact sheet). In 50 to 80% of all cases, the virus establishes a persistent infection, often leading to chronic liver disease, such as fibrosis, steatosis, cirrhosis. In 1 to 5% of persons with of patients with chronic infections liver cancer develops over a period of 20 to 30 years. 1 to 5% of infected persons may die. Long term effects are leading indications for liver transplants. Persons at risk for HCV infection might also be at risk for infection with Hepatitis B virus (HBV) or HIV. These infections potentiate the severeness of any single infection. Alcohol also accelerates disease progression.

HCV is transmitted by blood contacts from an infected person to an uninfected one, eg. through needle sharing, needle sticks, exposure to sharp objects for healthcare workers, from an infected mother to her baby during birth.

Current therapy comprises a combination of polyethylene glycol-conjugated alpha (peg) interferon and ribavirin or each component individually. The combination therapy can get rid of the virus in up to 50% for genotype 1 and in up to 80% for genotypes 2 and 3, but success rates are limited, and severe side effects as well as high costs restrict the use of this therapy.

It was recently demonstrated by the inventor the use of siDNA targeted to the HIV genome. This activates the RNase H of the virus and leads to silencing of the viral RNA (Matzen et al, Nature Biotechnol. 25, 669-674 (2007). The RNase H is a Hybrid-specific RNase which cleaves RNA in an RNA-DNA hybrid and was discovered by Moelling et al (Nature New Biology 234, 240-243 (1971)). We also observed that cellular RNase H-like activities such as RNase H1 and RNase H2a,b,c, and even Agonaute 2 contribute to siDNA-mediated silencing. Agonaute 2 (Ago2) is the enzyme known to induce siRNA-mediated silencing. We showed that it Ago2 is enzymatically related to RNases H and can induce siDNA-mediated silencing as well (Moelling et al. Cold Spring Harbor Symposium 71, Small RNAs, (2007)).

Furthermore, it could be shown by the inventor that siDNA is able to induce silencing of an oncogenic retrovirus, the Spleen Focus-Forming Virus and prevent infection, cause delay of disease progression and lead to increased survival time (Nature Biotechnol. 25, 669-674 (2007)). Furthermore the inventor showed in several cases that siDNA is superior to a single-stranded antisense effect (Jendis et al. AIDS Research and Human Retroviruses 12, 1161-1168 (1996), AIDS Research and Human Retroviruses 14, 999 - 1005, (1998), Moelling et al, FEBS Letters, 580, 3545-3550 (2006), Matzen et al. Nature Biotechnol. 25, 669-674 (2007)).

Thus the effect of siDNA has been demonstrated in several cases.

Recently siRNA has been described against HCV (Chevalier, Saulnier, Benureau, Flechet, Delgrange, Colbere-Garapin, Wychowski, Martin in: Mol.Ther. May 15, (2007): Inhibition of Hepatitis C virus Infection in Cell Culture by small interfering RNAs). The authors selected several siRNAs, some of them are strongly involved in tertiary RNA structures, which are not favored to be opened up by another RNA strand through siRNA.

The problem to be solved with the present invention is to present an effective antiviral therapeutic against Hepatitis C virus (HCV) infections.

The problem of the invention is solved by the features of the independent claims.

Therefore, siDNA oligonucleotides, capable of binding to one or more RNA target regions of Hepatitis C virus (HCV), as antiviral therapeutic, are object of the invention whereby the siDNA oligonucleotides are consisting of an antisense-strand homologous to the viral RNA target and a second strand, partially complementary to the antisense-strand forming a hairpin-loop structure. The siDNA oligonucleotides correspond to one or more HCV target regions of at least 20 nucleotides in length, whereby the antisense strand of siDNA is fully or almost fully homologous to the target HCV-RNA. Further, the second strand of the siDNA oligonucleotides is connected to the antisense strand through a thymidine linker, preferred - but not exclusively - 4 nucleotides in length, and whereby further the second strand is partially complementary to the antisense-strand and may be able to form triple helices by non-Watson-Crick base pairing with the viral RNA target strand.

Object of the invention is the use of siDNA as antiviral therapeutic. These are oligodeoxynucleotides consisting of an antisense-strand homologous to the viral RNA and a second strand, partially complementary to the antisense-strand. The siDNA is targeted to a conserved region of HCV, 20 to 25 nucleotides or longer, which is fully or almost fully homologous to the target RNA. A second strand is connected to the antisense strand through a Thymidine linker, e.g. 4 nucleotides in length. The second strand is partially complementary to the antisense-strand and may be able to form triple helices by non-Watson-Crick base pairing.

Also a preferred embodiment of the invention is the use of a combination of two or three siDNAs as described herein as antiviral therapeutic capable of binding to RNA target regions of HCV as pharmaceutical agent. Further preferred is the use of a cocktail of different siDNA oligonucleotides to target different HCV variants.

The siDNA will be applied to an infected cell or an infected individual with a transducing agent, whereby the transducing agent is selected from the following group: the virus itself, a replicating HCV particle which carries the siDNA into the cell during the process of infection, a liposome.

The siDNA oligonucleotides according to the invention are preferred as antiviral therapeutic as part of a pharmaceutical composition comprising these siDNA oligonucleotides capable of binding to RNA target regions of HCV.

### More detailed description of the invention

The invention is based on the cognition that a DNA strand has a thermodynamic preference to form an RNA-DNA hybrid over double-stranded DNA or double-stranded RNA. Thus the invention is based on the not expected result that siDNA is of high preference (in contrast to siRNA) to target RNA by forming RNA-DNA hybrids; siDNA is therefore of advantage and a preferred object of the invention. Furthermore siDNA acts against the newly infecting incoming viral RNA, 2 to 3 days before siRNA. Thus a virus infection can be prevented by siDNA only. In contrast thereto, interferon-induction is a risk for siRNA and reduced or absent for an RNA-DNA hybrid or double-stranded DNA. Furthermore, siDNA is preferred due to the fact that DNA is easier to synthesize and more stable. Also, it can be combined with siRNA.

The siDNA may also be a chimeric molecule, consisting of ribonucleotides and desoxyribonucleotides. However, the RNase H cleavage site needs to consist of a local RNA-DNA hybrid.

siDNA is also superior to a simple antisense oligodeoxynucleotide, because it is more stable during uptake by the cell and inside the cell and therefore more effective. It acts earlier than antisense DNA, and can target incoming RNA, while antisense DNA targets mRNA (Jekerle, V. et. al. J. Pharm. Sci. 8, 516-527 (2005)).

It was shown before that siDNA is targeting viral RNA before replication while siRNA is effective only late during replication targeting the mRNA (Westerhout,E.M. ter Brake,o. and Berkhout, B. Retrovirology 3,57-65 (2006)).

The invention takes advantage of this effect in silencing of HCV RNA. Using siDNA oligonucleotides is significantly more effective in contrast to siRNA. An unexpected advantage in conjunction with the present invention of siDNA *versus* siRNA is the targeting of incoming viral RNA before replication while siRNA is effective only late during replication targeting the mRNA. At this stage the RNA is highly amplified (e.g.1000fold) while the incoming RNA consists of a single copy. Viral RNA is destroyed within 8 to 14 hours post infection by 10fold reduction with no siRNA effect. At this timepoint the siRNA effect is undetectable. The siRNA needs to enter a multiprotein complex, RISC, to prepare the cleavage, a process which takes about 3 days. By then the virus has replicated efficiently. After 20 to 30 hours the reduction of viral RNA is similar in both cases, amounting to about 3- to 4fold reduction.

The RNA silencing inside the cell is achieved by the cellular RNases H, such as RNase H1 or RNase H2a,b,c. Also the Ago2, the siRNA-inducing silencing enzyme, is RNase H-like. These enzymes are located mainly in the nucleus but also in the cytoplasm. They are mainly responsible for removing RNA primers during DNA replication in the cell. The siDNA will lead to silencing of the viral RNA during early stages of replication and/or mRNA during late stages of replication, which requires higher doses. Treatment is performed by injection of siDNA into the blood stream or intraperitoneally. Depending on the stability of the compound the treatment can be repeated. Toxicity has not been detected in mouse studies (Matzen et al. Nature Biotech. 25,669-674(2007)).

As stated above, it could be shown by the inventor that siDNA is able to induce silencing of an oncogenic retrovirus, the Spleen Focus-Forming Virus and prevent infection, cause delay of disease progression and lead to increased survival time (Nature Biotechnology, June 3rd, 2007).The mechanism was due to the action of the viral RNase H. A contribution of cellular RNases H or related cellular enzymes is likely. New unpublished results show that a reporter plasmid can be targeted by an siDNA and that the gene expression is silenced by RNase H1 or RNase H2a. This was demonstrated by inactivation of these RNases H, which strongly reduces the silencing effects.

Preferred embodiments of the invention are described following:
G-tetrade formation may be beneficial consisting e.g. of GGXXGGXXXXGGG etc, whereby X represents other nucleotides than G, such as A, T or C. The siDNA oligonucleotides according to the invention contain G clusters to allow tetrade or tetramer formation or tetra-helices by sequences such as GGXXGGXXGG, up to 6Gs and 6 or 7X, whereby X is A, T or C. The hairpin-loop structure may consist preferably of up to 9 non-self-complementary sequences at the 3'- and 5'-ends, followed by 6base-pairing, 2non-pairing, 3pairing, 2non-pairing, 3pairing sequences, followed by 4Ts (see for proof of principle Moelling et al, FEBS Letters, 580, 3545-3550 (2006)).

Similar to siRNA a combination of two or three siDNAs may be targeted to different regions of the RNA genome or a cocktail may be designed to target different HCV variants. The preferred siDNA sequences are connected by a linker, preferably a thymidine linker, in particular a T4-linker.

siDNA can also be targeted to cellular mRNAs coding for proteins essential for HIV replication and indirectly prevent HVC replication.

Preferred according to the invention are the sequences for siDNAs as shown below which are also an object of the invention:

### Sequences:

HCV RNA (target A) [Seq.ID.1]
5' ACC CGG AAU UGC CAG GAC GAC CGG 3' 
siDNA (HCV)          [Seq.ID3 and Seq.ID2]
5' TGG GCC TTA TGC CAG CTC GAA AAG G -[TTTT] -
C CCG GTC GTC CTG GCA ATT CCG GGT 3'
HCV-RNA (target B) [Seq.ID.4]
5' CCC GGG AGG UCU CGU AGA CGU GCA C 3'

SIDNA (HCV)          [Seq.ID6 and Seq.ID5]
5'GGG CCC TCC TCT CGT TCA CGC ACA C -[TTTTT] -
G TGC ACG TCT ACG AGA CCT CCC GGG 3'

| Sequence | Sequence name | Sequence korr | Length |
|---|---|---|---|
| Seq.ID1 | RNA-HCV target A | 5′-ACC CGG AAU UGC CAG GAC GAC CGG G -3′ | 25 |
| Seq.ID2 | siDNA antisense A | 3′-TGG GCC TTA ACG GTC CTG CTG GCC C-5′ | 25 |
| Seq.ID3 | siDNA second strand A | 5′-TGG GCC TTA TGC CAG CTC GAA AAG G-3′ | 25 |
| Seq.ID4 | RNA-HCV target B | 5′-CCC GGG AGG UCU CGU AGA CGU GCA C-3′ | 25 |
| Seq.ID5 | siDNA antisense B | 3′-GGG CCC TCC AGA GCA TCT GCA CGT G-5′ | 25 |
| Seq.ID6 | siDNA second strand B | 5′-GGG CCC TCC TCT CGT TCA CGC ACA C-3′ | 25 |

The siDNA "antisense" strands and the siDNA "second strand" are linked via a thymidine (T4) linker.

The siDNA may be stabilized by base modifications e.g. thioates at the ends and/or in the center. The invention is not restricted to those kinds of base modifications. Other base modifications, i.e phosphorothioates, P-DNA, sugar-phopsphate modifications, Morpholinos, amidates, 2'-OMe, 2'-F, 2'-MOE, LNA and further are also preferred without limitation to those modifications.

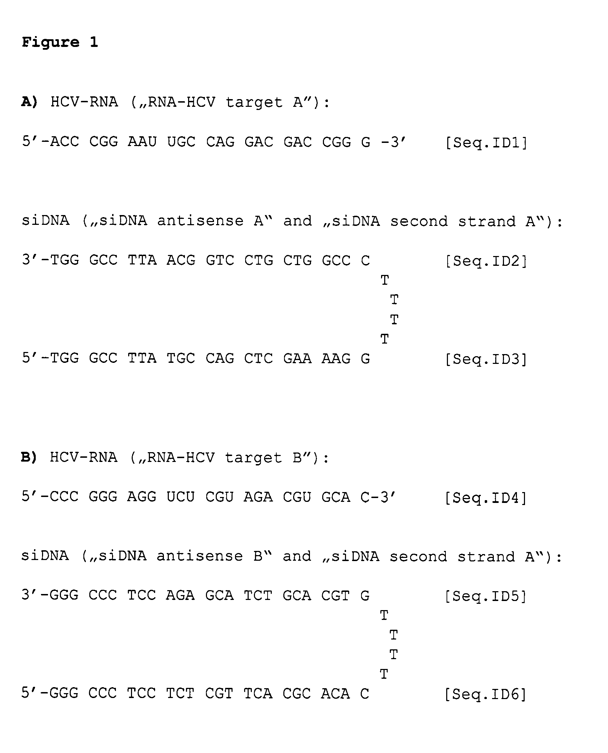

## Claims

1. siDNA oligonucleotides, capable of binding to one or more RNA target regions of Hepatitis C virus (HCV), as antiviral therapeutic, whereby the siDNA oligonucleotides are consisting of an antisense-strand homologous to the viral RNA target and a second strand, partially complementary to the antisense-strand.

2. siDNA oligonucleotides according to claim 1, whereby the siDNA molecule corresponds to one or more HCV target regions of at least 20 nucleotides in length, whereby the antisense strand of the siDNA is fully or almost fully homologous to the target HCV-RNA.

3. siDNA oligonucleotides according to one or more of the forgoing claims, whereby the second strand is connected to the antisense strand through a thymidine linker, preferred 4 nucleotides in length, and whereby the second strand is partially complementary to the antisense-strand and may be able to form triple helices by non-Watson-Crick base pairing with the viral RNA target strand.

4. siDNA oligonucleotides according to the preceding claims, whereby the siDNA oligonucleotides contain G clusters to allow tetrade or tetramer formation or tetra-helices by sequences such as GGXXGGXXGG, up to 6Gs and 6 or 7X, whereby X is A, T or C.

5. siDNA oligonucleotides according to the preceding claims, whereby the siDNA is stabilized by base modifications.

6. Use of siDNA, or a combination of two or three siDNAs according to the preceding claims, as antiviral therapeutic capable of binding to RNA target regions of HCV or different HCV variants, whereby different siDNA oligonucleotides may be combined in a cocktail as pharmaceutical agent.

7. Use according to claim 6, whereby the siDNA can also contain ribonucleotides, siDNA/RNA chimeras, or can be combined with siRNAs.

8. Use according to claim 6 or 7, whereby the siDNA will be applied to an infected cell or an infected individual with a transducing agent.

9. Use according to claim 8, whereby the transducing agent is selected from the following group: the virus itself, a replicating HCV particle which carries the siDNA into the cell during the process of infection, a liposome, transmembrane carriers or peptides.

10. Pharmaceutical composition comprising siDNA capable of binding to RNA target regions of HCV as antiviral therapeutic.
